Europäisches Patentamt

European Patent Office

Office européen des brevets

(19)

(11) Publication number: **0 087 087**
**A2**

# EUROPEAN PATENT APPLICATION

(12)

(21) Application number: 83101339.6

(22) Date of filing: 11.02.83

(51) Int. Cl.³: **C 12 N 5/00**
**A 61 K 35/12**

(30) Priority: 13.02.82 JP 21562/82

(43) Date of publication of application:
31.08.83 Bulletin 83/35

(84) Designated Contracting States:
BE CH DE FR GB IT LI NL SE

(71) Applicant: NIPPON SHINYAKU COMPANY, LIMITED
14, Kisshoin Nishinosho Monguchicho
Minami-ku Kyoto-shi Kyoto(JP)

(72) Inventor: Enomoto, Hiroshi
26-3-7-707 Babakemba Hashiri
Nagaokakyo-shi Kyoto-fu(JP)

(72) Inventor: Ozaki, Masakuni
7-25 Mitani, Terada
Joyo-shi Kyoto-fu(JP)

(72) Inventor: Watanabe, Hisao
1-6-353-9 Ninomaru-cho, Mukaijima
Fushimi-ku Kyoto(JP)

(72) Inventor: Matsuda, Masato
19-16 Seifucho
Otsu-shi Shiga Prefecture 520-02(JP)

(74) Representative: Wey, Hans-Heinrich, Dipl.-Ing. et al,
Patentanwälte Müller-Börner Wey & Körner
Widenmayerstrasse 49
D-8000 München 22(DE)

(54) A method of manufacturing biological active substance.

(57) Biologically active substances having anti-tumor activity which specifically act on cancer cells only and do not act on normal cells are produced by a method, in which cells derived from animals (including from human beings) or hybrid cells thereof are cultured and the resulting supernatant is purified.

Figure 3.

## A Method of Manufacturing Biologically Active Substances

The present invention relates to a method of manufacturing anti-tumor substances which do not act on normal cells but specifically act on cancer cells only, characterized in that, cells derived from animals (including human beings; this is the same throughout the specification) or hybrid cells thereof are cultured and the resulting supernatant fluid is purified.

Pharmaceutical agents which directly act on cancer cells and are used for the therapy of cancer such as, for example, mitomycin C, bleomycin, adriamycin, endoxan, 5-FU and other chemotherapeutics exhibit strong inhibitory action against cancer cells but they also act on normal cells, particularly on stem cells in bone marrow, and apt to cause side effects such as decrease in leucocytes and platelets and, as a result, patients are made predisposed and there are many cases of death due to infection and, consequently, there is a restriction in the use of such chemotherapeutics in clinical applications.

Non-specific immunotherapy in which the above side effects are eliminated has been widely used but, all of them possess high selective toxicity like penicillins against bacteria and, today, there is no drugs which are "to-the-point".

As the recent development of cell technology, monoclonal antibody against tumor associated antigens has become available and an attempt to destroy cencer cells selectively by the use of such antibody has been conducted. However, it is effective for specific cancer cells only and, therefore, there are some difficulty in tumor specificity and species specificity.

The present inventors have conducted extensive studies in order to find anti-tumor substances which do not exhibit species specificity, do not act on normal cells but act selectively on cancer cells only, and exhibit very little side effects such as shock to human beings and, by the use of cells of animal origin, the present inventors have accomplished the present invention.

The present invention is to manufacture biologically active substances which selectively act on cancer cells only, do not act on normal cells and exhibit anti-tumor activity by culturing cells derived from animal in a medium with or without serum for two to twenty four hours together with lipopolysaccharide (LPS) which is a kind of bacterial endotoxin or by culturing hybrid cells of such cells with myeloma for thirty six to seventy two hours.

Any cell can be used in the present invention so far as it derives from animal and it has an ability of producing the present invention substances. Examples of such cells are M1 or Mm1 which is myeloid leukemic cell line derived from mice, J-774.1 or P-388D1 which is monocytic leukemic cell line derived from mice, and the like.  However, since the present invention aims a therapy for human beings, it is preferred to use cells derived from human beings such as, for example, J-111, THP-1, U-937 and the like which are monocytic leukemic cell line derived from human beings.

It is preferred that those cells are subjected to growth by a method commonly used for culturing normal animal cells such as, for example, that subjected to a growth at 37°C in 5% $CO_2$-95% air using RPMI 1640 medium containing 10% calf serum and then subjected to the present invention.

In order to obtain the present invention substances from cells cultured under such conditions, it is preferred to add 10 ng to 1 microgram/ml of bacterial endotoxin such as LPS, lipid A or liposome thereof, lectin such as concanavalin A, interferone inducer such as polyinosine-cytidine and the like and to culture at 37°C for two to twenty four hours in a mixture of 5% $CO_2$ and 95% air.

When 2-mercaptoethanol is added to a serum-free medium, the present invention substances with high titer can be obtained.

Further, animals immunized with BCG, Corynebacterium, etc. are stimulated with LPS etc, the resulting peritoneal exudate cell or spleen cells or tumor cell line  producing the present invention substances is made into a hybrid cell using P3-X63Ag strain or BW 5147 strain which is  myeloma  cell and polyethylene glycol, the said hybrid cell having an ability of producing the present invention substances with stimulation of LPS or the like, and the said hybrid cell is cultured whereupon large quantities of the present invention substances without LPS in the supernatant fluid.

In order to use the culture solution containing the present invention substances obtained under the above conditions as pharmaceuticals, a process which is a combination from ultrafiltration, dialysis, ion exchange method, affinity chromatography, gel filtration, electrophoresis, and the like is applied. In order to purify more effectively and in less cost, affinity chromatography using specific antibody as an adsorbing material may be applied.

It is desirable to use tumor cells of animal origin in obtaining the present invention substances. Among such cells, those grow as monolayer are not suitable for production in large scale. There is an advantage that the cell lines such as THP-1, U-937, M1, Pu-5-1.8, and the like are possible in large amount culture even in suspending state. In addition, it is obvious such strains are not infected by virus and this is advantageous in such a point that the products are aimed for the therapy of human beings. In the production of the present invention substances from those cells, it is desired to add five to ten percent of serum in most cases but, even when no serum is added to a medium, sufficient amount of the present invention substances can be obtained. This is far more advantageous in isolation and purification of the product as compared with the method in which the product is obtained from serum.

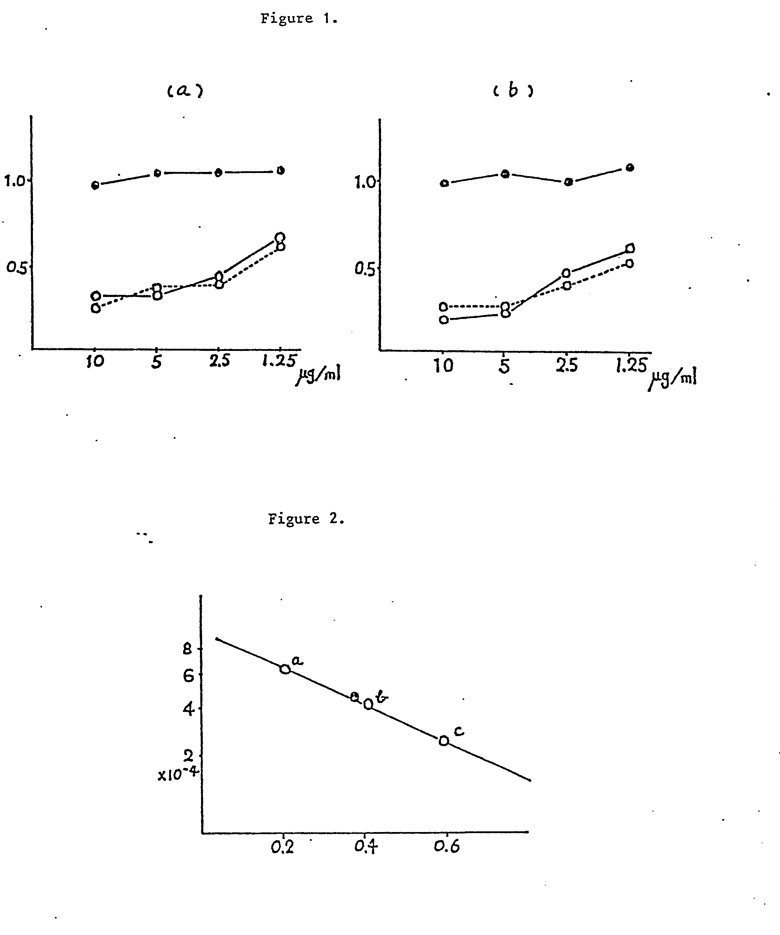

Using L-929 strain (fibroblast derived from mice) and HeLa-S3 strain (carcinoma of uterine corvix derived from human beings) as target cells and mouse embryonic cell as normal target cell, cytotoxicity of the present invention substances is studied according to a method by Frederic C. Kull, et al (Journal of Immunology, volume 126, page 1279, 1981) in vitro. Thus, target cells (400,000 cells/well) are placed in a multiplate (Limbro) with 24 holes for tissue culture, the present invention substances diluted are simultaneously added thereto, cultured at 37°C for forty eight hours in a mixture of 5% $CO_2$ and 95% air, and the status of cells (whether dead or alive) is judged by way of uptaking of dyes (Neutral Red). The results are as given in Figures 1a and b that the present invention substances do not exhibit toxicity to normal cells at all but exhibit toxicity to cancer cells of human beings and mice without species specificity.

The present invention substances also show anti-tumor effect in vivo. Thus, 1,000,000 cells/mouse of sarcoma S-180 (mouse allogeneic·tumor) are transplanted subcutaneously to ddY strain mouse. Also, 1,000,000 cells/mouse of Meth-A (mouse syngenic tumor) are transplanted subcutaneously to BALB/c mouse. On the seventh day after the transplantations, 10 micrograms/mouse of the present invention substances are administered to each of the above systems intraperitoneally once and, five days later, the same amount is administered to each of them. On the fifteenth day after the transplantations, the tumor parts are taken out by cutting and their weights are measured. The results are as given in Table 1 that the present invention substances show marked regression to both kinds of tumors. In addition, complete regression is observed in some animals.

The present invention substances are stable against the heating of 56°C for thirty minutes, show molecular weight of 40,000 to 50,000 according to a method of SDS-polyacrylamide gel electrophoresis (hereinafter abbreviated as "SDS-PAGE"), positive to PAS dyeing, and stable at pH 5 to 10. They show high specificity against cancer cells and do not exhibit species specificity as observed in interferone. They may be obtained from cells derived from human beings and have characteristics that they have little danger of side effects such as a shock due to heteroprotein. They show marked anti-tumor effect even in vivo and are excellent and ideal anti-tumor substances far more better than conventional anti-tumor substances hitherto known. Thus they are stable and can be expected to show marked effect as pharmaceuticals.

The present invention is further illustrated by the following examples.

Example 1.

THP-1 strain cells derived from human monocytic leukemia are suspended in RPMI 1640 medium (Nissui Seiyaku Co) containing 80 micrograms/ml of streptomycin (Meiji Seika Co) and 10% fetal calf serum (GIBCO) to prepare a suspension of 500,000 cells/ml. The suspension is cultured at 37°C for seven days

in 5% $CO_2$-95% air under a suspending status. Grown cells are collected by centrifugation with cooling (1000 rotations per minute; for five minutes), re-suspended in the above medium to prepare a suspension containing 5,000,000 cells/ml, lipopolysaccharide 055,B5 (DIFCO) derived from Escherichia coli is added thereto to make it 1·nanogram to 10 micrograms per milliliter, cultured at 37°C for two to four hours in

5% $CO_2$-95% air, and subjected to centrifugation with cooling to afford a supernatant fluid.

  This is desalted and concentrated by way of ultrafiltra-

tion (using a machine of Amicon), subjected to an ion exchange

chromatography using DEAE-Sephacel (Pharmacia) treated with

0.05M Tris hydrochloric acid buffer (pH 7.6), eluted with 0 to 0.3M gradients of NaCl, active fractions are collected, dialyzed, and lyophilized. then passed through Blue-Sepharose CL-6B (Pharmacia) affinity chromatography treated with 0.05M Tris hydrochloric acid buffer (pH 7.2) to remove albumin therefrom, subjected to gel filtration using Sephacryl-S 200 (Pharmacia) treated with the above buffer, and an active substance (about 42,000 molecular weight by SDS-PAGE; positive to PAS dyeing) is obtained.

- 8 -

Example 2.

THP-1 strain cells are cultured and grown for seven days by the same method as described in Example 1, then re-suspended in RPMI 1640 medium without serum to prepare a suspension of 5,000,000 cells/ml, and treated the same as Example 1 to give a substance of Example 1.

When 0.005M 2-mercaptoethanol is added thereto, twice to five times stronger titer of substance of Example 1 is obtained.

Example 3.

Active substances obtained in Examples 1 and 2 are subjected to several subcutaneous immunizations to rabbits together with Freund's complete adjuvant, the resulting antiserum (30 ml) is dialyzed to 0.1M phosphate buffer (pH 7.0), added to a column of protein A-Sepharose CL.4B (Pharmacia) treated with the same buffer so that IgG fraction is selectively adsorbed thereto, then eluted with 1M acetic acid, the eluate is dialyzed to 0.1M sodium carbonate buffer (pH 8.3), and lyophilized to give 340 mg of IgG fraction.

The fraction (100 mg) dissolved in 0.1M sodium carbonate buffer (pH 8.3) is then added to 20 ml of CNBr-Sepharose 4B (Pharmacia) treated with 1mM HCl, stirred for two hours at room temperature, let stand overnight at 4°C, the resulting gel suspension is packed in a column of 1 x 20 cm, well washed with 0.5M NaCl/0.1M sodium carbonate buffer (pH 8.3), then 25 ml of 1M ethanolamine/0.1M sodium carbonate buffer (pH 8.0) is passed thereinto, and equilibrated by washing well with 0.1M sodium carbonate buffer (pH 8.3).

Concentrated supernatant fluids of the culture obtained in Examples 1 and 2 is dialyzed to 0.1M sodium carbonate buffer (pH 8.3), added to the affinity column combined with antibody which is obtained hereinabove, and eluted with 0.17M glycine hydrochloric acid buffer (pH 5.3) to afford the same substances as Examples 1 and 2.

Example 4.

M1 cells (which are myeloid leukemic cells derived from mice) are suspended in Dulbecco's modified MEM medium (Nissui Seiyaku Co) containing 80 micrograms/ml of streptomycin (Meiji Seika) and 10% horse serum (Flow) to prepare a suspension of 1,000,000 cells/ml, then 10 micrograms/ml of lipopolysaccharide is added thereto, and cultured at $37^{o}$ C in 5% $CO_2$-95% air for three days under suspending conditions.

Then the cells are collected by centrifugation with cooling (1000 rotations/min; for five minutes), then re-suspended in the same medium as above to prepare a suspension of 5,000,000 cells/ml, 1 nanogram to 10 micrograms/ml of lipopolysaccharide 0.55,B5 (DIFCO) derived from Escherichia coli is added thereto, cultured at 37°C in 5% $CO_2$-95% air for twenty four hours, and treated the same as in Example 1 to afford an active substance whose molecular weight is about 45,000 by SDS-PAGE.

Example 5.

M1 cells are cultured for three days by the same method as in Example 4, re-suspended in Dulbecco's MEM medium without serum to prepare 5,000,000 cells/ml suspension, and then treated with the same way as in Example 1 to afford the substance of Example 4.

When 0.005M 2-mercaptoethanol is added thereto, a substance of Example 4 with twice to thrice stronger titer is obtained.

Example 6.

u-937 strain (histiocytic leukemia cells derived from human beings) is suspended in RPMI 1640 medium (Nissui Seiyaku Co) containing 80 micrograms/ml of streptomycin (Meiji Seika) and 10% fetal calf serum (GIBCO) to prepare a suspension of 100,000 cells/ml. This is cultured at 37°C for three days in 5% $CO_2$-95% air under suspending conditions, and treated the same way as in Example 1 to afford an active substance of about 48,000 molecular weight (according to SDS-PAGE).

Example 7.

U-937 strain cells are cultured for three days by the same way as in Example 6, then re-suspended in RPMI 1640 medium without serum to prepare a suspension of 5,000,000 cells/ml, and treated the same way as in Example 1 to afford a substance of Example 6.

When 0.005M 2-mercaptoethanol is added thereto, the substance of Example 6 with two to five times stronger titer is obtained.

- 11 -

Example 8.

Inactivated Corynebacterium parvum (1 mg/mouse) is administered intraperitoneally to BALB/c mice (male; six weeks age), 10 micrograms/mouse of lipopolysaccharide 055 B5 (DIFCO) derived from Escherichia coli is administered intraperitoneally after one week, cells coming to abdominal cavity are collected two hours later, treated with P3 x 63Ag strain (which is myeloma cells derived from BALB/c) and polyethylene glycol to afford hybrid cells which are then selected using HAT selection medium, subjected to cloning with a soft agar method, the supernatant fluid of culture of hybrid cells which can grow in RPMI 1640 medium (5% fetal calf serum added thereto) producing the present invention substances is purified by the same method of Example 1, and active substance with about 40,000 molecular weight is obtained.

4. Brief Explanation of Drawings:

Figure 1 shows cell toxicity in vitro of the present invention substance obtained in Example 1 against (a) normal mouse embryonic mice cells(closed circles) and L-929 cancer cells ( open circles) and against (b) normal mouse embryonic cells (closed circles) and HeLa-S3 cancer cells (open circles). The vertical axis and horizontal axis show dye uptake (OD value) and concentrations, respectively. Solid lines and dashed lines show the case serum added medium is used and that serum-free medium is added, respectively. It is apparent from the figures that the present invention substances are specifically active
on cancer cells only and they have no species specificity.

Figure 2 shows the relation of molecular weight of standard protein with migration in SDS-PAGE. The vertical and
horizontal axises show molecular weight and migration, respectively. Letters a, b and c represent bovine serum albumin
(BSA), oval albumin (OVA) and chymotrypsinogen, respectively.
Closed circle shows a position of the present invention substance obtained in Example 1 and wherefrom the molecular weight
of the present invention substance can be estimated.

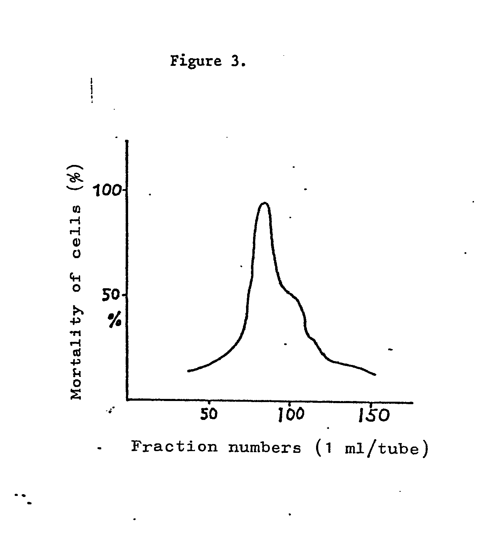

Figure 3 is a gel filtration patterns of the present invention substance obtained in Example 1 by Sephacryl-S 200. The
vertical and horizontal axises show mortality of cells and
fraction numbers (1 ml/tube), respectively.

Table 1. Anti-tumor effect against S-180 and Meth-A

| | Numbers of Animals | Mean tumor weight (g ± SE) | |
|---|---|---|---|
| | | ( S-180 ) | (Meth-A) |
| ( Control) | 10 | 0.63 ± 0.07 | 0.74 ± 0.05 |
| Substance of Example 1 (10 micrograms/mouse) (intravenous injection) | 10 | 0.23 ± 0.03 | 0.33 ± 0.04 |

We Claim:

(1) A method of manufacturing biologically active substances having anti-tumor activity which specifically acts on cancer cells only and does not act on normal cells, characterized in that, cells derived from animals or hybrid cells thereof are cultured and the resulting culture supernatant is purified.

(2) A manufacturing method according to Claim 1 in which the cells are derived from human beings.

(3) A manufacturing method according to Claim 1 or Claim 2 in which the culture is conducted using a serum free medium.

(4) A manufacturing method according to Claim 3 in which the culture is conducted using a serum free medium to which 2-mercaptoethanol is added.

(5) A manufacturing method according to Claims 1 to 4 in which the culture is conducted by using a culture liquid to which liposaccharide or lipid A or liposome, lectin or interferone inducer thereof is added.

(6) A manufacturing method according to Claims 1 to 5 in which the purification is conducted by an affinity chromatography using a specific antibody as an adsorbing agent.

Figure 1.

(a)                              (b)

Figure 2.

Figure 3.

Fraction numbers (1 ml/tube)